# EUROPEAN PATENT SPECIFICATION

(11) **EP 2 408 442 B1**
(45) Date of publication and mention of the grant of the patent: **17.08.2016**
(21) Application number: 10715741.4
(22) Date of filing: 22.03.2010
(51) Int. Cl.: A61K 31/12, A61K 9/48, A61P 29/00

(54) **PHARMACEUTICAL COMPOSITION PRESENTING ANTI-INFLAMMATORY PROPERTIES**
PHARMAZEUTISCHE ZUSAMMENSETZUNG MIT ENTZÜNDUNGSHEMMENDEN EIGENSCHAFTEN
COMPOSITION PHARMACEUTIQUE À PROPRIÉTÉS ANTI-INFLAMMATOIRES

(30) Priority: 20.03.2009 EP 09155800; 31.12.2009 EP 09181041
(43) Date of publication of application: 25.01.2012
(73) Proprietor: Bioxtract S.A., 1348 Louvain-la-Neuve (BE)
(72) Inventor: PRIEM, Fabian, B-1332 Genval (BE); JACQUEMOND-COLLET, Ingrid, B-5001 Belgrade (BE)
(74) Representative: Pronovem
(86) International application number: PCT/EP2010/053719
(87) International publication number: WO 2010/106191

(56) References cited:
- WO-A-02/074295
- WO-A-2006/130679
- WO-A2-2007/103435
- US-A- 4 263 333
- US-A1- 2006 045 928
- DATABASE WPI Section Ch, Week 200739 17 January 2007 (2007-01-17) Thomson Scientific, London, GB; Class B04, AN 2007-404940 XP002587396 J. CHEN, B. GAO, J. XIONG: "Curcumin liquid preparation for preventing or treating cancer, cardiovascular and cerebrovascular disease comprises curcumin, emulsifier, antioxidant, isotonic regulator, stabilizer, pH regulator and water." & CN 1 895 239 A (UNIV NO 2 MILITARY MEDICAL PLC) 17 January 2007 (2007-01-17)
- HSU-CHIH CHEN ET AL: "The encapsulation of curcumin in Micelles" BIOENGINEERING CONFERENCE, 2005. PROCEEDINGS OF THE IEEE 31ST ANNUAL N ORTHEAST HOBOKEN, NEW JERSEY, USA APRIL 2-3, 2005, PISCATAWAY, NJ, USA,IEEE, 2 April 2005 (2005-04-02), pages 275-276, XP010799185 ISBN: 978-0-7803-9105-5
- HAITO K ET AL: "Preparation of aqueous turmeric pigment formulation for coloring acidic food and drink, cosmetics, and quasi drug, involves mixing curcumin solution substantially dissolved in polysorbate as solubilizer with aqueous solvent" WPI / THOMSON,, 1 January 1900 (1900-01-01), XP002449958 & JP 2005 185237 A (SANEI GEN FFI INC) 14 July 2005 (2005-07-14)
- TONNESSEN H HJORTH: "Solubility and stability of curcumin in solutions containing alginate and other viscosity modifying macromolecules - Studies of curcumin and curcuminoids. XXX" PHARMAZIE, DIE, GOVI VERLAG, ESCHBORN, DE, vol. 61, no. 8, 1 August 2006 (2006-08-01) , pages 696-700, XP008089665 ISSN: 0031-7144

## Description

### Field of the invention

The present invention relates to a new pharmaceutical, a nutraceutical (or a food (or a feed) additive) or a functional food (or feed) composition presenting an improved formulation of plant extracts to increase the bio-availability of the active components present in this composition, and its use in the treatment and/or the prevention of inflammation and an inflammation-related syndromes or disorders.

### State of the Art

Numerous agents identified from fruits and vegetables can interfere with several cell-signaling pathways. The agents include curcumin (turmeric), but also resveratrol (present in red grapes, peanuts and berries), genistein (present in soybean), diallyl sulfide (present allium), S-allyl cysteine (present in allium), allicin (present in garlic), lycopene (present in tomato), capsaicin (present in red chilli), diosgenin (present in fenugreek), 6-gingerol (present in ginger), ellagic acid (present in pomegranate), ursolic acid (present in apple, pears, prunes), silibinin (present in milk thistle), anethol (present in anise, camphor, and fennel), catechins (present in green tea), eugenol (present in cloves), indole-3-carbinol (present in cruciferous vegetables), limonene (present in citrus fruits), beta carotene (present in carrots), and dietary fibers (present in many vegetables and fruits).

In vitro, the cell-signaling pathways inhibited by curcumin seems to include one or more of the following biological factors NF-κB, AP-1, STAT3, Akt, Bcl-2, Bcl-XL, caspases, PARP, IKK, EGFR, HER2, JNK, MAPK, COX2, and 5-LOX.

Curcumin is the principal curcuminoid of the popular Indian curry spice turmeric. The curcuminoids are polyphenols and are responsible for the yellow color of turmeric.

Extensive work has been done in attempts to establish the biological activities and pharmacological actions of curcumin, at least *in vitro* (including experiments carried-out on cultured cell). Curcumin may be beneficial for its antitumor (cancer), antioxidant, antiarthritic, anti-ischemic, anti-diabetic, anti-amyloid, and antiinflammatory properties, in addition to possible benefits in cardiovascular diseases, neurological diseases and in Crohn's disease.

However, at neutral and basic pH, curcumin is rapidly degraded *in vitro.*

In addition, when eaten, little (if no) curcumin is absorbed: curcumin undergoes avid intestinal metabolism and its intestinal absorption is low, precluding reaching blood concentrations of curcurmin compatible with effects demonstrated *in vitro.*

It is postulated in the art that blood concentrations of curcumin (and its derivatives) of about 0.5 µM to about 6 µM are safe, and compatible with the advantageous effects measured *in vitro.*

Besides the metabolism, rapid systemic elimination of curcumin may further contribute to the rather limited clinical effect.

To improve its bioavailability (at the plasma and tissue levels), numerous approaches have been undertaken. These approaches may involve the use of adjuvants, like piperine. Indeed, piperine interferes with glucuronidation, a physiological mechanism (occurring in the liver and small intestine) of modification of compounds foreign to an organism's normal biochemistry (such as drugs and poisons), in order to increase their water solubility and their excretion.

Co-supplementation with 20 mg of piperine (extracted from black pepper) may increase the plasma concentration of curcumin.

However, due to its effects on drug metabolism, (i.e., inhibition of glucuronidation) piperine should be taken cautiously (if at all) by individuals taking other medications.

Alternatively, dissolving curcumin in hot water prior to ingestion, or in warm oily liquids, appears to increase its bioavailability, but this fact is not well established and heat instability of curcumin is frequently postulated.

Heat- and light- stability of curcumin appear to be increased by its encapsulation into cyclodextrin (Szente et al., 1998, Journal of inclusion Phenomena and Molecular Recognition in Chemistry, 32, 81-89).

Other stable metabolites of curcumin, such as tetrahydrocurcumin are used in topical application and possibly found *in vivo,* but it is not clear if they provide the same therapeutic activity as curcumin.

The presence of acids increases the stability of curcumin, but decreases its solubility. Conversely basic environment increase the curcumin solubility, but at the expense of a sharply reduced stability.

Therefore, caution must be taken when formulating more bio-available curcumin.

Compositions containing curcumin and alginate or curcumin and gelatine have a solubility increased by >10⁴ fold at pH=5. However, curcumin was not stabilized towards hydrolytic or photolytic degradation.

The use of liposomal curcumin or curcumin nanoparticles or curcumin phospholipid complex may be beneficial.

For instance, nanoparticles comprising curcumin have a size of less than 100 nanometers on average demonstrated comparable to superior efficacy compared to free curcumin in human cancer cell line models. However, actual *in vivo* absorption has not been demonstrated with these nanoparticles.

WO2007/101551 describes a method to increase the bioavailability of curcumin that involves a simple procedure creating a complex with soy phospholipids, however the obtained plasma concentration of curcumin using this formulation only reached 0.033 micromolar.

WO2006/130679 describes compositions containing chemicals having functional carbonyl groups for the treatment of allergic rhinitis.

It is suggested in this international patent application WO2006/130679 that curcumin (that bears carbonyl groups) may possibly react *in vitro* with histamine through the formation of Schiff bases involving a carbonyl group of curcumin and the amine group of histamine, and it is therefore speculated that compounds having functional carbonyl groups might be of value for a treatment of allergic rhinitis (*in vivo*).

In one example of the patent application WO2006/130679, a commercial mixture of curcumin and polysorbate is used.

However, in the patent application WO2006/130679, the commercial composition of curcumin with polysorbate was demonstrated to be ineffective in reacting with histamine.

Furthermore, this prior art teaches that too acid mixtures should be avoided, since no Schiff base can be formed at acidic pH.

Finally, no *in vivo* data are disclosed in the patent application WO2006/130679.

New methods resulting in enhanced *in vivo* effect of curcumin are therefore required to bring this promising natural product to the forefront of therapeutic agents for treatment of various human diseases.

### Summary of the invention

The present invention provides a composition comprising a sufficient amount of curcumin, a sufficient amount of an emulsifier and a sufficient amount of citric acid, the said composition being for use as a medicament, wherein the sufficient amount of the curcumin is comprised between 2 mg and 200 mg, wherein either the emulsifier has a low viscosity at 25°C of 0.100-0.800 N.s.m⁻² (Pa.s), combined to a medium to high hydrophilic-lipophilic balance, the medium hydrophilic-lipophilic balance being 6-10 and the high hydrophilic-lipophilic balance being 11-25, and a sufficient amount of said low viscosity emulsifier is comprised between 400 mg and 700 mg, the sufficient amount of curcumin is comprised between 10 mg and 100 mg, the ratio of curcumin to the low viscosity emulsifier is comprised between 5:95 and 15:85 (w:w), the citric acid being present at a concentration comprised between 1 mg and 10 mg, the emulsifier being Polysorbate 80; or the emulsifier has a high viscosity at 50°C comprised between 75.0 and 175.0 N.s.m⁻² (Pa.s), combined to a medium to high hydrophilic-lipophilic balance, the medium hydrophilic-lipophilic balance being 6-10 and the high hydrophilic-lipophilic balance being 11-25, and a sufficient amount of said high viscosity emulsifier is comprised between 200 mg and 500 mg, the sufficient amount of curcumin is comprised between 5 mg and 50 mg, the ratio of curcumin to the high viscosity emulsifier is comprised between 5:95 and 15:85 (w:w), the citric acid being present at a content comprised between 1 mg and 10 mg, the emulsifier being Polysorbate 60.

Preferably, the composition is further encapsulated in gelatine capsule.

Advantageously, the composition further comprises between 3% (w:w) and 30% (w:w) of an essential oil comprising more than 50% of terpen compounds.

Preferably, the composition comprises the low viscosity emulsifier, wherein the sufficient amount of said low viscosity emulsifier is comprised between 600 mg and 700 mg.

In a preferred embodiment, the composition comprises the low viscosity emulsifier, wherein the sufficient amount of curcumin is comprised between 30 mg and 50 mg.

Advantageously, the composition comprises the low viscosity emulsifier, wherein the ratio of curcumin to the low viscosity emulsifier is comprised between 5:95 and 12:88 (w:w).

Preferably, the composition comprises the low viscosity emulsifier, wherein the citric acid is present at a concentration comprised between 2 mg and 5 mg.

In a preferred embodiment, the composition comprises the high viscosity emulsifier, wherein the sufficient amount of said high viscosity emulsifier is comprised between 300 mg and 500 mg.

Advantageously, the composition comprises the high viscosity emulsifier, wherein the sufficient amount of curcumin is comprised between 10 mg and 30 mg.

Preferably, the composition comprises the high viscosity emulsifier, wherein the ratio of curcumin to the high viscosity emulsifier is comprised between 5:95 and 12:88 (w:w).

In a preferred embodiment, the composition comprises the high viscosity emulsifier, wherein the citric acid is present at a concentration comprised between 1 mg and 5 mg.

Advantageously, the composition comprises the high viscosity emulsifier, further comprising a dryer selected from the group consisting of cellulose, methylcellulose, hydroxypropylcellulose and SiO₂.

Preferably, the composition is intended for use in the treatment or the prevention of inflammation or inflammation-related diseases.

In a preferred embodiment, the composition is intended for use in the treatment or the prevention of osteoarthritis.

The present invention thus provides a new composition and methods to obtain and use this composition that does not present the drawbacks of the products of the state of the art, especially a composition and methods that improves the bioavailability of curcumin in mammal blood, especially in human blood.

Furthermore, the invention provides a composition that comprises only food ingredients or elements that are compatible for an administration as a food (or feed) additive or in a functional food (or feed) composition.

Finally, the invention provides such composition that can be used for improving the treatment and/or the prevention of inflammation or inflammation-related syndromes or disorders, while possibly requiring lower administration doses than the known composition of the state of the art.

The inventors have found a method to increase the bio-availability of an active compound being curcumin, for an administration to an animal (preferably a mammal, including a human) by providing this curcumin in a composition containing an edible (and advantageously food compatible) emulsifier.

Advantageously, the composition of the invention may further comprise an essential oil (preferably a (water-free) mixture of monoterpens and/or sesquiterpens).

By providing (administrating) such composition to an animal, this curcumin (including curcumin derivatives, preferably selected from the group consisting of dihydrocurcumin, tetrahydrocurcumin and glucuronides and sulphate derivatives of curcumin, dihydrocurcumin and tetrahydrocurcumin) concentration value in this animal blood (preferably a mammal blood, including the human) is of at least 0.5 µM.

Advantageously, in the method of the invention, from about 20 mg to about 500 mg of this curcumin (preferably from about 40 mg to about 120 mg of curcumin) present in this composition is given (administrated) (orally) on a daily basis (to an adult human) to reach blood concentration of this curcumin and curcumin derivatives (including also glucuronides and sulphate derivatives of curcumin, as well as possibly tetrahydrocurcumin) of at least (about) 0.5 µM (in an adult human), preferably of at least (about) 1 µM.

Alternatively, about 100 mg to about 250 mg of curcumin, preferably, about 150 mg (alternatively about 168 mg) of curcumin present in the composition of the invention is given (administrated) (orally) on a daily basis to reach blood concentration of this curcumin and curcumin derivatives (including also glucuronides and sulphate derivatives of curcumin, as well as possibly tetrahydrocurcumin) of at least (about) 1.5 µM (in an adult human), preferably of at least (about) 2 µM.

Therefore, an aspect of the present invention relates to a pharmaceutical, a nutraceutical (also called food (or feed) additive composition) or a functional food or functional feed composition comprising (or consisting of):
- possibly an adequate (pharmaceutical or food (feed) compatible) carrier or diluent and
- a sufficient amount of curcumin (E100), of an emulsifier and possibly of an essential oil (a (water-free) mixture of more than 50% terpen compounds: monoterpens and/or sesquiterpens);
this composition being preferably used as a medicament, more preferably used for a treatment and/or a prevention of inflammation or an inflammation-related syndrome or disorder, preferably the treatment and/or the prevention of osteoarthritis.

Advantageously, in the (pharmaceutical) composition according to the invention, the sufficient amount of curcurmin (present in one capsule) is comprised between about 2 mg and about 200 mg, preferably between about 2 mg and about 100 mg, more preferably between about 20 mg and about 50 mg, preferably about 42 mg; alternatively about 30 mg.

By inflammation-related syndrome or disorder, it is meant a mammal (human) disease caused by or presenting an inflamed status, including inflammation.

More precisely, inflammation-related syndromes or disorders encompass cancer, cardiovascular diseases, preferably selected from the group consisting of atherosclerosis and ischemic heart (Ischemia) disease, asthma, auto-immune diseases, chronic inflammation, chronic prostatitis, glomerulonephritis, hyper sensitivities, inflammatory bowel disease, pelvic inflammatory disease, reperfusion injury, auto-immune diseases or allergic disease, such as rhinitis, osteoarthritis (rheumatoid arthritis), transplant rejection, vasculitis, abdominal obesity and/or neurodegenerative diseases, such as Alzheimer disease, Parkinson disease or Huntington disease.

These syndromes or disorders include also allergic reactions formerly known as type 1 hyper sensitivity (that are the result of an inappropriate immune response (inflammation-related disorder) triggering inflammation) and other hyper sensitivity reactions (type 2 and type 3) mediated by antibody reactions and that induces inflammation by attracting leukocytes, which damage surrounding tissues. These syndromes and disorders include also inflammatory myopathies caused by the immune system inappropriately attacking component of muscle leading to muscle inflammation (that may occur in conjunction with other immune disorders, such as systemic sclerosis and including dermatomyositis, polymyositis and inclusion body myositis). These syndromes and disorders include also high level of several inflammations relating markers, preferably selected from the group consisting of IL-6, IL-8 and TNF-alpha that are associated with obesity.

Prolonged inflammation (inflammation-related disorder), known as acute or chronic inflammation leads to a progressive shift of the type of cells which are present at a site of inflammation and is characterized by simultaneous destruction and healing of tissues from the inflammatory process that could lead known chronic diseases syndromes or disorders, preferably selected from the group consisting of obesity, diabetes mellitus, cardio- and cerebrovascular diseases like hypertension or ischemia, auto-immune diseases (including diseases of inflammatory origin like arthritis or lupus), brain diseases (including neuro-degenerative diseases, like Alzheimer disease, Parkinson disease, Huntington disease, multiple sclerosis, depression or schizophrenia), asthma, systemic sclerosis, allergies and cancer, one or all the above identified diseases being treated and/or prevented by the composition of the invention.

Osteoarthritis is the most preferred disease to be treated and/or prevented by the composition of the invention.

Another consequence of inflammation-related disorders is pain that is also to be treated and/or prevented by the composition of the invention.

Diseases involving pain in connective tissues, such as fibromyalgia, are also a syndrome or a disorder treated and/or prevented of the present invention.

Therefore, in the present description, the applicant will use for the same effect, the words "chronic inflammation", "chronic inflammation associated disease" or "inflammatory disorders" that constitute a large and related group of syndromes and disorders collectively named inflammation-related syndrome or disorder, which underlies a variety of mammal (human) diseases.

Preferably, the pharmaceutical composition of the present invention may further comprises an acid component, preferably an organic acid, more preferably selected from the group consisting of citric acid (E330), malic acid (E296), acetic acid (E260), tartaric acid (E334), lactic acid (E270), alginic acid (E400) or a mixture thereof.

Advantageously, the pharmaceutical composition of the present invention is combined with (encapsulated in) an adequate carrier, preferably a capsule, bead, pill or tablet, that is preferably food compatible, such as gelatine (capsule), that may be more preferably coated with an (food compatible) element that renders this composition resistant to gastric digestion in the stomach, more preferably this element being E904 (Shellac).

The present invention relates also to a pharmaceutical composition, nutraceutical or functional food/feed composition (including a food/feed additive), being preferably a non-aqueous solution and comprising (or consisting of):
- possibly an adequate (pharmaceutical or food compatible) carrier or diluent and
- a sufficient amount of curcumin, a sufficient amount of an emulsifier and a sufficient amount of citric acid and possibly a sufficient amount of an essential oil (a (water-free) mixture of monoterpens and/or sesquiterpens), preferably for use as a medicament and more preferably for use in the treatment and/or the prevention of inflammation, inflammation-related syndromes or disorders, more preferably in the treatment and/or prevention of osteoarthritis (rheumatoid arthritis).

Advantageously, the composition (either in a form of a non aqueous solution or a solid composition and/or in a capsule) of the invention comprising a sufficient amount of curcumin, a sufficient amount of an emulsifier and possibly of a sufficient amount of an acid, is taken orally and is a per os (pharmaceutical or neutraceutical or functional food/feed) composition.

Preferably, between about 2 mg and about 200 mg of curcumin, more preferably between about 2 mg and about 100 mg of curcumin, more preferably between about 20 mg and about 50 mg of curcumin, a sufficient amount of one emulsifier and possibly a sufficient amount of an acid are formulated in one capsule according to the present invention, this capsule being the pharmaceutical composition of the invention.

Advantageously 1, 2, 3, 4, 5, 6, 7, 8, 9 or up to 10 capsules, preferably 1 to 6 capsules, more preferably 2 to 4 capsules of the invention are taken by a subject on a daily basis.

By 'about', it is preferably meant a variation of plus or minus 10%. For instance about 5 % means every real number from 4.5% to 5.5%.

Advantageously, this composition is suitable to be taken orally, once, twice or thrice a day.

The oral composition of the invention may further comprise a sufficient amount of an essential plant oil.

Preferred essential oils according to the invention are selected from the group consisting of Agar oil, Ajwain oil, Anise oil, Balsam oil, Parsley oil, Bergamot oil, Cardamom seed oil, Carrot seed oil, Chamomile oil, Calamus oil, Cinnamon oil, Citronella oil, Fennel seed oil, Fenugreek oil, Frankincense oil, Galangal oil, Geranium oil, Ginger oil, Grapefruit oil, Henna oil, Juniper berry oil, Lavender oil, Lemongrass oil, Melissa oil, Mint oil, Myrrh oil, Orange oil, Oregano oil, Orris oil, Peppermint oil, Pine oil, Rosehip oil, Sage oil, Schisandra oil, Spikenard, Tarrangon oil, Tea tree oil, Thyme oil, Turmeric oil, Valerian oil, Yarrow oil and Zedoary oil, being more preferably Turmeric oil.

Advantageously, in the composition of the invention, from about 3% (w:w) to about 30% (w:w), preferably from about 5% (w:w) to about 15% (w:w), more preferably about 7% (w:w) of essential oils is present (added (to the others elements of composition of the invention)).

In the pharmaceutical (or nutraceutical) composition of the invention, preferably from about 25 ml to about 200 ml, more preferably about 50 ml of (water-free) essential oils may be added.

By essential oils, it is meant volatile and liquid (but non-aqueous) aroma compounds from natural sources, usually plants, with a poor solubility in water.

Essential oils are usually prepared by extraction techniques, preferably selected from the group consisting of distillation (including steam distillation), cold pressing, extraction (maceration) and/or solvent extraction.

The essential oils of the invention comprise more than 50% (consist essentially) of terpen (-related) compounds, such as monoterpens and/or sesquiterpens and are preferably water-free.

By "essentially consisting of terpen (-related) compounds", it is meant that at least 50% (on a molar basis), preferably at least 70%, more preferably at least 80% of the essential oils or all the essential oils are made of terpen (-related) compounds and are preferably water-free.

Terpens are derived biosynthetically from units of isoprene. Monoterpens consist of two isoprene units and sesquiterpens consist of three isoprene units. To the isoprene (monoterpens and/or sesquiterpens) backbone, additional hydrolysis and/or rearrangements and oxidations provide the final terpens compound of an essential oil.

Alternatively, the most abundant molecules of an essential oil (terpens compounds from essential oils) may be used in the composition of the invention, instead of a complex essential oil, for instance terpens (-related) compounds, preferably selected from the group consisting of ar-Turmerone, Turmerone, Curlone, ar-Curcumene and p-Cymene and a mixture thereof.

Advantageously, several emulsifiers are present and combined (mixed) in the composition of the invention, provided their mean HLB, viscosity, content and ratio of content is kept within the ranges disclosed hereafter.

Preferred emulsifiers according to one embodiment of the invention have a low viscosity and good water solubility.

Advantageously, the emulsifier(s) present in the composition according to the present invention has (have) a low viscosity (25°C) of about 0.100-0.800 Pa.s, combined to a medium (6-10) to high (11-25) hydrophilic-lipophilic balance (HLB), being preferably Polysorbate 80.

Advantageously, in the composition (in the form of a non aqueous solution) according to the invention, a sufficient amount of a low viscosity emulsifier(s) is comprised between about 200 mg and about 700 mg, more preferably between about 400 mg and about 700 mg, still more preferably between about 600 mg and about 700 mg, preferably between about 600 mg and about 650 mg.

Advantageously, in the composition (in the form of a non aqueous solution) according to the invention, a sufficient amount of curcurmin is comprised between about 5 mg and about 200 mg, preferably between about 10 mg and about 100 mg, more preferably between about 30 mg and about 50 mg, more preferably about 40 mg (alternatively about 42 mg).

Preferably, the ratio of curcumin to the low viscosity emulsifier(s) is comprised between about 1:99 and about 30:70 (w:w), more preferably between about 5:95 and about 15:85 (w:w), still more preferably between about 5:95 and about 12:88 (w:w), still more preferably of about 6:94 (w:w).

Advantageously in the (non aqueous solution) composition according to the invention, the acid component (preferably citric acid) is present at a content comprised between about 0.5 mg and about 35 mg, more preferably between about 1 mg and about 10 mg, more preferably between about 2 mg and about 5 mg, more preferably about 3 mg (alternatively, preferably about 3.5 mg).

Possibly, the (non aqueous solution) composition according to the invention may further comprise a sufficient amount of essential oil(s) (monoterpens and/or sesquiterpens).

Alternatively, in another embodiment of the invention, the emulsifier(s) present in the (solid) composition according to the present invention has (have) a high viscosity (50°C) comprised between about 75.0 and about 175.0 Pa.s, combined to a medium (6-10) to high (11-25) hydrophilic-lipophilic balance (HLB), this emulsifier being preferably Polysorbate 60.

Advantageously, in the (solid) composition according to the invention, a sufficient amount of the high viscosity emulsifier(s) is comprised between about 100 mg and about 700 mg, more preferably between about 200 mg and about 500 mg, still more preferably between about 300 mg and about 500 mg, and is more preferably of about 300 mg (or alternatively of about 260 mg).

Advantageously, in the (solid) composition according to the invention, a sufficient amount of the curcurmin is comprised between about 2 mg and about 100 mg, preferably between about 5 mg and about 50 mg, more preferably of between about 10 mg and about 30 mg, more preferably about 16 mg.

Preferably, the ratio of curcumin to the high viscosity emulsifier(s) present in the composition is comprised between about 1:99 and about 20:80 (w:w), more preferably between about 5:95 and about 15:85 (w:w), still more preferably between about 5:95 and about 12:88 (w:w), still more preferably of about 6:94 (w:w).

Advantageously in the (solid) composition according to the invention, the acid component is present at a concentration (content) comprised between about 0.25 mg and about 35 mg, more preferably between about 1 mg and about 10 mg, more preferably between about 1 mg and about 5 mg, more preferably 1.5 mg.

Possibly, the (solid) composition according to the invention may further comprise a sufficient amount of an (or a mixture of) essential oil(s) (monoterpens and/or sesquiterpens).

Advantageously, the (solid) composition comprises sufficient amount of curcumin, of (a) high-viscosity emulsifier(s), of an acid and of a dryer.

The dryer present in the composition is any (inert and food compatible) substance able to adsorb (and therefore to provide a solid aspect like a powder) the high-viscosity emulsifier(s) and being preferably selected from the group consisting of cellulose, methylcellulose, hydroxypropylcellulose (and combinations thereof), and SiO₂.

The preferred dryer according to the invention is SiO₂.

Advantageously, the amount of the dryer (preferably SiO₂ particules) comprised in the composition is between about 50 mg and about 400 mg, preferably between about 100 mg and about 300 mg, more preferably about 120 mg.

Preferably, this dryer (preferably SiO₂) is added to the pharmaceutical composition at 1:10 to about 5:10, more preferably at about 3:10 (w:w).

Preferably, the SiO₂ particules added to the composition of the invention are made of a silica of high absorptive capacity.

Preferably, the SiO₂ particules added to the composition of the invention is made of a silica of a low particle size, with an average diameter ranging from about 5 µm to about 100 µm, possibly sold under the name Sipernat 50S.

Preferably, the SiO₂ of the invention is non-derivatized.

Another aspect of the present invention is a method for the treatment of inflammatory disease by the step of administrating a sufficient amount of the composition of the invention to a mammal subject (preferably a human patient), possibly suffering from one of the above mentioned diseases, syndromes and disorders.

More preferably in the method of the invention, from about 20 mg to about 500 mg of curcumin (preferably from about 40 mg to about 170 mg of curcumin; alternatively, from about 40 mg to about 120 mg) is administrated on a daily basis to this mammal subject (preferably to an adult human patient) to reach blood concentration of this curcumin and curcumin derivatives to a concentration value of at least (about) 0.5 µM (in an adult human).

A last aspect of the invention is a method to obtain the composition of the invention comprising (consisting of) the steps of:
- preparing or obtaining substantially pure curcumin,
- dissolving the substantially pure curcumin in an adequate amount of emulsifier, preferably with an organic acid,
- optionally adding a sufficient amount of an (or a mixture of) essential oils (monoterpens and/or sesquiterpens),
- optionally mixing the composition consisting of this curcumin and of this emulsifier onto a dryer and crushing particles,
- filling a (gelatine) capsule with this composition comprising (consisting of) this curcumin and this emulsifier and possibly this dryer into,
- possibly obtaining an enteric coating (with addition of E904 layer(s)) of this (gelatine) capsule and
- recovering the composition of the invention.

Preferably, the (gelatine) capsule allows to package from about 700 mg to about 1000 mg of the (liquid) composition of the invention. Preferably, the (gelatine) capsule allows to package of about 400 mg of the (solid) composition of the invention.

### Detailed description of the invention

### Definitions

Curcumin is also known as [1,7-bis(4-hydroxyl-3-methoxyphenyl)-1,6-heptadiene-3,5-dione, as diferuloylmethane or as E100 food additive.

The present invention encompasses curcumin, including its keto and enol forms, but preferably not the chemically-derived forms of curcumin nor di-tetra- or hexa-hydrocurcumin.

Polysorbate 80, E433, Tween®80 or PEG 20 Sorbitan monooleate is an emulsifier.

Polysorbate 60, E435, Tween®60, PEG 20 Sorbitan monostearate is an emulsifier.

By viscosity, it is meant dynamic viscosity, expressed in Pa*s.

By emulsifier, it is meant edible compounds that are amphiphilic and therefore have the potential to reduce the interfacial tension between an hydrophobic compound (such as curcumin) and water by adsorbing at the liquid-liquid interface. The emulsifiers of the present invention may assemble into aggregates, such as micelles that can encapsulate the hydrophobic compound of the invention.

Surprisingly, the inventors found that acid mixtures of curcumin with the emulsifiers and possibly with essential oils (monoterpens and/or sesquiterpens) according to the invention result into improved stability of curcumin and into a sustained solubility in duodenal conditions (e.g. in a phosphate buffer at pH 6.8, 37°C), bio-availability and clinical effect.

### Examples

### Example 1 :

Commercial curcumin having a purity of at least 90% (the inventors preferably selected curcumin batches having a purity of at least 95% or 96% and possibly close to 100%) is used. These lots are obtained after extraction with ethanol and re-crystallization using methanol.

### Preparation of the liquid extract

The curcumin preparation is preferably made using curcumin powder with fine granulometry (having the preferred and advantageous granulometry comprised between about 100 and about 200 µm). Firstly, the citric acid crystals (having an advantageous granulometry below 150 µm) are mixed with curcumin. The resulting mixture is stirred into Polysorbate 80. After heating to 30°C for adequate homogenisation, this complete mixture is mixed for 45 min. Then the whole mixture is milled with a three-roll-mill (preferably Coball mill) and closing aerated with nitrogen to remove present air. The preparation is then encapsulated shortly in gelatine capsules, preferably about 700 mg per capsule. The inventors used non-coated capsules and also enteric coated capsules with addition of E 904 (SHELLAC).
The concentration of pure curcumin is preferably 6%, with 0.5% citric acid completed to 100% with Polysorbate 80.

### Example 2:

The preparation is made as in example 1, but using a high viscosity emulsifier, preferably Polysorbate 60. SiO₂ is then added to a final percentage of 10% to 60%, preferably 30% to 40% max (more preferably about 30%) in a planetary mixer or in a high sheer blender, until obtaining a homogenous and fluid powder. This powder can be compressed into a tablet or filled into hard shell capsules (preferably 400 mg per capsule).
The concentration of pure curcumin in the final composition is preferably of 4%, with 0.35% citric acid and a final concentration of SiO₂, preferably of 30%.
All percentages are on a weight by weight (w:w) basis).

### Example 3 :

Solubility of curcumin in the liquid composition of Example 1 is measured by agitating 1.4 g (equivalent to the posology of 2 capsules of 700 mg per day) of this preparation (containing 84 mg of curcumin) in 900 ml of phosphate buffer pH 6.8, for 1 hour at 37°C.
In comparison, the same amount of curcumin (84 mg) is solubilised in phosphate buffer pH 6.8, for 1 hour at 37°C. After 1 hour, the solution is filtered on a 0.2 µm filter and the solubilized curcumin is quantified.
Solubilized curcumin in the liquid composition of Example 1: 35% of the initial dose.

Curcumin in composition of Example 1 is 8750 times more soluble than curcumin without polysorbate and citric acid.

Solubility of curcumin in the powder composition of example 2 is measured by agitating 1.2 g (equivalent to the posology of 3 capsules of 400 mg per day) of this preparation (containing 48 mg of curcumin) in 900 ml of phosphate buffer pH 6.8, for 1 hour at 37°C.

In comparison, the same amount of curcumin (48 mg) is solubilised in phosphate buffer pH 6.8, for 1 hour at 37°C.
After 1 hour, the solution is filtered on a 0.2 µm filter and the solubilized curcumin is quantified.

Solubilized curcumin in composition of Example 2: 30% of the initial dose.

Curcumin in composition of Example 2 is 6000 times more soluble than curcumin without polysorbate and citric acid.

### Example 4 :

### Composition:

Curcumin preparation is made as in example 1 using curcumin powder with fine granulometry (100-200 µm). Firstly, the citric acid crystals (having an advantageous granulometry below 150 µm) are mixed with curcumin. The resulting mixture is stirred into Polysorbate 80 and Turmeric Essential Oil, extracted from rhizomes of *Curcuma longa* L. by supercritical extraction. It contains min. 60% of total turmerones (Aromatic-turmerones, alpha-turmerone and beta-turmerone). Hydrodistillation or steam distillation may be also applied to extract said essential oil. After heating to 30°C, the complete mixture is mixed for 45 min. Then the whole mixture is milled with a Coball Mill (three-roll-mill preferably) and closing aerated with nitrogen. The preparation is then encapsulated shortly in gelatine capsules, preferably enteric coated, preferably 700 mg per capsule.

The resulting composition is made of 6% (w:w) curcumin, 0.5% (w:w) citric acid and 7.14% (w:w) turmeric essential oil completed to 100% with Polysorbate 80 (86.36%; w:w).

Solubility:

Solubility of curcumin in the liquid composition of example 4 is measured by agitating 1.4 g (equivalent to the posology of 2 capsules of 700 mg per day) of this preparation (containing 84 mg of curcumin) in 900 ml of phosphate buffer pH 6.8, for 1h at 37°C.
In comparison, the same amount of the curcumin preparation (84 mg) of Example 1 is solubilised in phosphate buffer pH 6.8, for 1h at 37°C.

After 1h, the solution is filtered on a 0.2 µm filter and the solubilized curcumin is quantified. Solubilized curcumin from Example 1: 35% of the initial dose. Solubilized curcumin from Example 4 (with the addition of essential oil): 50% of the initial dose.

The inventors repeated the same experiment but with the replacement of turmeric essential oils by the same amount of Peppermint essential oil and obtained the same improved solubility over the curcumin composition comprising only an emulsifier.

As controls, the inventors mixed curcumin with essential oils and measured only a poor solubility of curcumin, close to the solubility of pure curcumin.

The inventors conclude that curcumin in a composition comprising an emulsifier and supplemented with essential oil is 12.500 times more soluble than a curcumin composition with essential oils but without emulsifier, and 1.5 more soluble than a curcumin composition with emulsifier and citric acid (without essential oils), although the amount of emulsifier is reduced in the curcumin composition with citric acid, emulsifier and essential oils.

### Example 5 :

### Oral Bioavailability Test

The inventors firstly evaluated the acute toxicity of the formulation of the invention. The inventors noticed no toxicity.

A pre-defined high dose (5000 mg/kg) of the formulation of Example 1 was given in a single oral dose to rats. Animals were daily observed for at least 14 days.

Formulation of Example 1 was then studied in human volunteers to evaluate the relative oral bioavailability. Twenty-four healthy individuals volunteers aged between 18 and 55 years were selected for the study. The following treatments were administered as single doses (once a day): 1 "enteric-coated" capsule (6 males and 6 females) and 2 "enteric-coated" capsules (6 males and 6 females). "Enteric-coated" capsule is a gelatine capsule, coated with Shellac, and filled with 700 mg of a mixture of curcumin (6%; 42 mg), citric acid (0.5%; 3.5 mg) and as emulsifier polysorbate 80. Blood samples were taken at zero hour and periodically at one-hour or half-hour intervals for 12 hours (0, 0.5, 1, 1.5, 2, 3, 4, 6, 8 and 12 hours after dosing). At each sample collection, 6 ml of blood were sampled in heparin tubes. After centrifugation, plasma was transferred into two labelled tubes. The first tube was spiked with internal standard (clenbuterol) and extracted with methanol and acetonitrile. The extract was analysed by UPLC-MS/MS on a BEH-C18 column (100 x 2.1 mm, particles size 1.7 µm) using water + formic acid and acetonitrile + formic acid as solvent. The eluant flow rate was 0.6 ml/min and detection was achieved using an electrospray ionisation source (ESI) operating in positive ion mode. Efficiency of the extraction procedure for recovering curcumin from blood samples was determined by recovery of curcumin upon normal blood samples. The curcumin recovery was calculated at three different concentration levels with at least 5 replicates by comparing peak area for curcumin with those obtained by direct injection of reference solutions in triplicates at the same concentration levels.

No quantifiable trace of non-metabolized curcumin was observed irrespective of the subject, the time point and the dose.

The blood samples were then analysed after hydrolysis of potential conjugated metabolites of curcumin by means of β-glucuronidase and arylsulfatase. Samples were assayed for curcumin and its conjugates after incubating the plasma samples with the enzymes β-glucuronidase and sulfatase. For these assays, plasma samples (200 µL) were mixed with 20 µL of 1 M acetate buffer (pH 5.5), 5 µL of β-glucuronidase and arylsulfatase solution, and 20 µL of internal standard solution (clenbuterol; 2 µg/ml). The samples were incubated at 37°C for 3 h (determined after a test run). The samples were extracted with 1 mL of ethyl acetate/methanol (95:5; v/v). After centrifugation, the supernatant was evaporated gently to dryness under nitrogen stream at ambient temperature and reconstituted in 100 µL of methanol and 50 µL of water and 7.5 µL were injected into the UPLC system.

After this treatment with β-glucuronidase and arylsulfatase, the inventors noticed Cmax values of curcumin in blood of 196 ng/ml and of 333 ng/ml (Table 1).

**Table 1: Summary statistics for curcumin derivatives (measured as curcumin after hydrolysis of curcumin derivatives); pharmacokinetics parameters per dose**

| | | AUC(0-inf) (ng × h/ml) | AUC(0-t) (ng × h/ml) | Cmax (ng/ml) | Tlag (h) | Tmax (h) | T1/2 (h) |
|---|---|---|---|---|---|---|---|
| 1 capsule | n | 11 | 12 | 12 | 12 | | 11 |
| | Mean (SD) | 888.974 (571.7139) | 724.603 (479.7565) | 195.742 (136.6893) | 3.167 (2.1567) | 5.000 (2.9233) | 2.697 (0.8636) |
| | CV % mean | 64.312 | 66.210 | 66.831 | 68.11 | 58.465 | 32.018 |
| | Geo-mean | 658.000 | 539.480 | 145.981 | 2.4626 | 4.219 | 2.584 |
| | CV% geo-mean | 119.7742 | 116.4587 | 110.6014 | 103.23 | 72.3560 | 30.7865 |
| | Median | 1057.820 | 661.815 | 176.600 | 3.000 | 4.000 | 2.370 |
| | [Min ; Max] | [117.86 ; 1900.67] | [87.80 ; 1704.48] | [29.70 ; 480.90] | [0.50 ; 8.00] | [1.00 ; 12.00] | [1.73 ; 4.53] |

| 2 capsules | n | 10 | 12 | 12 | 12 | 12 | 10 |
|---|---|---|---|---|---|---|---|
| | Mean (SD) | 1938.345 (1090.9189) | 1330.797 (945.9549) | 333.033 (252.9666) | 2.792 (1.6020) | 5.042 (2.2203) | 3.148 (0.9816) |
| | CV % mean | 56.281 | 71.082 | 75.958 | 57.38 | 44.040 | 31.181 |
| | Geo-mean | 1625.572 | 957.501 | 241.916 | 2.368 | 4.364 | 3.015 |
| | CV% geo-mean | 75.1433 | 123.9572 | 112.0137 | 67.794 | 71.8203 | 31.6947 |
| | Median | 1594.145 | 1231.100 | 269.950 | 2.500 | 6.000 | 2.860 |
| | [Min ; Max] | [540.89 ; 3804.92] | [135.30 ; 3338.43] | [45.50 ; 847.80] | [1.00 ; 6.00] | [1.00 ; 8.00] | [1.78 ; 4.66] |

Different papers have demonstrated that such low dosages of oral (not formulated) curcumin (42 or 84 mg of curcumin) were not bioavailable at all, even under metabolised forms (glucuronides or sulfates of curcumin).

The inventors further confirmed this aspect by giving the same amount of curcumin in a 700 mg capsule with no emulsifier. They did not measured curcumin in blood, even after treatment with β-glucuronidase and arylsulfatase, as above.

The inventors then compared enteric-coated capsules with uncoated capsules comprising the same amount of curcumin and emulsifier.

After treatment with β-glucuronidase and arylsulfatase as above, the inventors noticed similar maximal blood values with a Cmax value of curcumin in blood of about 300-350 ng/ml in both cases (2 capsules were given).

Overall, the inventors conclude that the composition they developed is bio-available and measured mainly curcumin derivatives.

### Example 6 :

### Clinical trials

The inventors then evaluated the compositions of the invention (preferably the composition of Example 1) in the treatment of osteoarthritis.

The inventors selected 280 patients suffering for knee osteoarthritis and randomized them (50:50) in a placebo and in a treated group. The treated group received 4 capsules (one capsule: curcumin, 42 mg; citric acid, 3.5 mg and as emulsifier polysorbate 80) of the invention per day, for 14 days, while the placebo group received 4 (visually) identical capsules with no curcumin.

The study was double blind, performed in several sites, and monitored by experts rheumatologists.

The patients were selected following inclusion criteria such as age (40-80 years old), presence of knee osteoarthritis associated with pain (using Visual analogue Scale; VAS; typically patients have had a VAS>50 mm for the last 24 hours while not taking analgesics), and exclusion criteria, such as the co-existence of other rheumatoid diseases or of other medication (except paracetamol).

The Lequesne index (global index of pain and patient's physical capacities) was measured at Day 0 and at Day 14, as well as their pain (VAS) and the imaging of their arthritis-affected knee.

The patients were requested to note their evaluation of pain on the Likert scale (0: no pain at all; 10: maximal pain) on a daily basis and, if they took paracetamol, the quantity (max. 4g per day, except at days 13-14 where paracetamol was not allowed). During the study, the patients were further requested not to take antiinflammatory or analgesic compounds (other than paracetamol).

The inventors further monitored any adverse event that occurred.

At Day 14, the estimation of pain (VAS), Lequesne index, and all the data collected by the patients (patient's estimation of pain on Likert scale, quantity of paracetamol that was taken) were collected.

When all the patients had completed the 14-days treatment, the inventors unblinded the data.

There was no difference in the adverse events in both the placebo and the treated groups.

The main difference over the placebo group was the reduction in pain (VAS and/or Likert scale) and/or in Lequesne score, as measured by the physician and/or by the patients from Day 0 and Day 14 and/or of the consumption of paracetamol at days 1 to 12.

Overall, the inventors concluded that patients receiving capsules according to the invention (containing curcumin, organic acid and an emulsifier) have a reduced pain and improved capacities. They thus conclude that the formulation they developed is efficient in treating osteoarthritis and/or its symptoms.

## Claims

1. A composition comprising a sufficient amount of curcumin, a sufficient amount of an emulsifier and a sufficient amount of citric acid, the said composition being for use as a medicament, wherein the sufficient amount of the curcumin is comprised between 2 mg and 200 mg,
wherein either the emulsifier has a low viscosity at 25°C of 0.100-0.800 N.s.m⁻² (Pa.s), combined to a medium to high hydrophilic-lipophilic balance, the medium hydrophilic-lipophilic balance being 6-10 and the high hydrophilic-lipophilic balance being 11-25, and a sufficient amount of said low viscosity emulsifier is comprised between 400 mg and 700 mg, the sufficient amount of curcumin is comprised between 10 mg and 100 mg, the ratio of curcumin to the low viscosity emulsifier is comprised between 5:95 and 15:85 (w:w), the citric acid being present at a concentration comprised between 1 mg and 10 mg, the emulsifier being Polysorbate 80;
or the emulsifier has a high viscosity at 50°C comprised between 75.0 and 175.0 N.s.m⁻² (Pa.s), combined to a medium to high hydrophilic-lipophilic balance, the medium hydrophilic-lipophilic balance being 6-10 and the high hydrophilic-lipophilic balance being 11-25, and a sufficient amount of said high viscosity emulsifier is comprised between 200 mg and 500 mg, the sufficient amount of curcumin is comprised between 5 mg and 50 mg, the ratio of curcumin to the high viscosity emulsifier is comprised between 5:95 and 15:85 (w:w), the citric acid being present at a content comprised between 1 mg and 10 mg, the emulsifier being Polysorbate 60.

2. The composition for use according to the claim 1 being further encapsulated in gelatine capsule.

3. The composition for use according to claims 1 or 2, further comprising between 3% (w:w) and 30% (w:w) of an essential oil comprising more than 50% of terpen compounds.

4. The composition for use according to any of the preceding claims comprising the low viscosity emulsifier, wherein the sufficient amount of said low viscosity emulsifier is comprised between 600 mg and 700 mg.

5. The composition for use according to any of the preceding claims comprising the low viscosity emulsifier, wherein the sufficient amount of curcumin is comprised between 30 mg and 50 mg.

6. The composition for use according to any of the preceding claims comprising the low viscosity emulsifier, wherein the ratio of curcumin to the low viscosity emulsifier is comprised between 5:95 and 12:88 (w:w).

7. The composition for use according to any of the preceding claims comprising the low viscosity emulsifier, wherein the citric acid is present at a concentration comprised between 2 mg and 5 mg.

8. The composition for use according to any of the claims 1 to 3 comprising the high viscosity emulsifier, wherein the sufficient amount of said high viscosity emulsifier is comprised between 300 mg and 500 mg.

9. The composition for use according to any of the claims 1 to 3 or 8 comprising the high viscosity emulsifier, wherein the sufficient amount of curcumin is comprised between 10 mg and 30 mg.

10. The composition for use according to any of the claims 1 to 3 or 8 to 9 comprising the high viscosity emulsifier, wherein the ratio of curcumin to the high viscosity emulsifier is comprised between 5:95 and 12:88 (w:w).

11. The composition for use according to any of the claims 1 to 3 or 8 to 10 comprising the high viscosity emulsifier, wherein the citric acid is present at a concentration comprised between 1 mg and 5 mg.

12. The composition for use according to any of the claims 1 to 3 or 8 to 11 comprising the high viscosity emulsifier, further comprising a dryer selected from the group consisting of cellulose, methylcellulose, hydroxypropylcellulose and SiO₂.

13. The composition according to any of the preceding claims for use in the treatment or the prevention of inflammation or inflammation-related diseases.

14. The composition for use according to claim 13, wherein the inflammation-related disease is osteoarthritis.

## Patentansprüche

1. Zusammensetzung, die eine ausreichende Menge an Kurkumin, eine ausreichende Menge eines Emulgators und eine ausreichende Menge an Zitronensäure umfasst, wobei die Zusammensetzung für eine Verwendung als Medikament vorgesehen ist, wobei die ausreichende Menge des Kurkumins zwischen 2 mg und 200 mg beträgt,
wobei entweder der Emulgator bei 25 °C eine geringe Viskosität von 0,100 bis 800 Ns/m⁻² (Pa.s) aufweist, kombiniert zu einem mittleren bis hohen hydrophilen/lipophilen Gleichgewicht, wobei das mittlere hydrophile/lipophile Gleichgewicht 6-10 ist und das hohe hydrophile-lipophile Gleichgewicht 11-25 ist, und eine ausreichende Menge des Emulgators mit geringer Viskosität zwischen 400 mg und 700 mg beträgt, die ausreichende Menge an Kurkumin zwischen 10 mg und 100 mg beträgt, das Verhältnis von Kurkumin zum Emulgator mit geringer Viskosität zwischen 5:95 und 15:85 (Gew./Gew.) beträgt, wobei die Zitronensäure in einer Konzentration zwischen 1 mg und 10 mg vorhanden ist, wobei der Emulgator Polysorbate 80 ist;
oder der Emulgator eine bei 50 °C hohe Viskosität zwischen 75,0 und 175,0 Ns/m⁻² (Pa.s) aufweist, kombiniert zu einem mittleren bis hohen hydrophilen/lipophilen Gleichgewicht, wobei das mittlere hydrophile/lipophile Gleichgewicht 6-10 ist und das hohe hydrophile-lipophile Gleichgewicht 11-25 ist, und eine ausreichende Menge des Emulgators mit hoher Viskosität zwischen 200 mg und 500 mg beträgt, die ausreichende Menge an Kurkumin zwischen 5 mg und 50 mg beträgt, das Verhältnis von Kurkumin zum Emulgator mit hoher Viskosität zwischen 5:95 und 15:85 (Gew./Gew.) beträgt, wobei die Zitronensäure in einem Gehalt zwischen 1 mg und 10 mg vorhanden ist, wobei der Emulgator Polysorbate 60 ist.

2. Zusammensetzung zur Verwendung nach Anspruch 1, die des Weiteren in einer Gelatinekapsel verkapselt ist.

3. Zusammensetzung zur Verwendung nach Anspruch 1 oder 2, die des Weiteren zwischen 3 % (Gew./Gew.) und 30 % (Gew./Gew.) eines essentiellen Öls umfasst, das mehr als 50 % Terpenverbindungen umfasst.

4. Zusammensetzung zur Verwendung nach einem der vorstehenden Ansprüche, die den Emulgator mit geringer Viskosität umfasst, wobei die ausreichende Menge des Emulgators mit geringer Viskosität zwischen 600 mg und 700 mg beträgt.

5. Zusammensetzung zur Verwendung nach einem der vorstehenden Ansprüche, die den Emulgator mit geringer Viskosität umfasst, wobei die ausreichende Menge an Kurkumin zwischen 30 mg und 50 mg beträgt.

6. Zusammensetzung zur Verwendung nach einem der vorstehenden Ansprüche, die den Emulgator mit geringer Viskosität umfasst, wobei das Verhältnis von Kurkumin zum Emulgator mit geringer Viskosität zwischen 5:95 und 12:88 (Gew./Gew.) beträgt.

7. Zusammensetzung zur Verwendung nach einem der vorstehenden Ansprüche, die den Emulgator mit geringer Viskosität umfasst, wobei die Zitronensäure in einer Konzentration zwischen 2 mg und 5 mg vorhanden ist.

8. Zusammensetzung zur Verwendung nach einem Ansprüche 1 bis 3, die den Emulgator mit hoher Viskosität umfasst, wobei die ausreichende Menge des Emulgators mit hoher Viskosität zwischen 300 mg und 500 mg beträgt.

9. Zusammensetzung zur Verwendung nach einem der Ansprüche 1 bis 3 oder 8, die den Emulgator mit hoher Viskosität umfasst, wobei die ausreichende Menge an Kurkumin zwischen 10 mg und 30 mg beträgt.

10. Zusammensetzung zur Verwendung nach einem der Ansprüche 1 bis 3 oder 8 bis 9, die den Emulgator mit hoher Viskosität umfasst, wobei das Verhältnis von Kurkumin zum Emulgator mit hoher Viskosität zwischen 5:95 und 12:88 (Gew./Gew.) beträgt.

11. Zusammensetzung zur Verwendung nach einem der Ansprüche 1 bis 3 oder 8 bis 10, die den Emulgator mit hoher Viskosität umfasst, wobei die Zitronensäure in einer Konzentration zwischen 1 mg und 5 mg vorhanden ist.

12. Zusammensetzung zur Verwendung nach einem der Ansprüche 1 bis 3 oder 8 bis 11, die den Emulgator mit hoher Viskosität umfasst, die des Weiteren ein Trocknungsmittel umfasst, das aus der Gruppe ausgewählt ist, bestehend aus Zellulose, Methylzellulose, Hydroxypropylzellulose und SiO₂.

13. Zusammensetzung nach einem der vorstehenden Ansprüche zur Verwendung bei der Behandlung oder Vorbeugung von Entzündungs- oder entzündungsbedingten Krankheiten.

14. Zusammensetzung zur Verwendung nach Anspruch 13, wobei die entzündungsbedingte Krankheit Osteoarthritis ist.

## Revendications

1. Composition contenant une quantité suffisante de curcumine, une quantité suffisante d'un émulsifiant et une quantité suffisante d'acide citrique, ladite composition étant destinée à une utilisation en tant que médicament, la quantité suffisante de la curcumine étant comprise entre 2 mg et 200 mg,
dans laquelle soit l'émulsifiant a une faible viscosité à 25°C de 0,100 à 0,800 N.s.m⁻² (Pa.s), en combinaison avec un rapport hydro-lipophile moyen à élevé, le rapport hydro-lipophile moyen étant de 6 à 10 et le rapport hydro-lipophile élevé étant de 11 à 25, et une quantité suffisante dudit émulsifiant à faible viscosité est comprise entre 400 mg et 700 mg, la quantité suffisante de curcumine est comprise entre 10 mg et 100 mg, le rapport de la curcumine à l'émulsifiant à faible viscosité est compris entre 5:95 et 15:85 (p/p), l'acide citrique étant présent à une concentration comprise entre 1 mg et 10 mg, l'émulsifiant étant le Polysorbate 80 ;
soit l'émulsifiant a une viscosité élevée à 50°C, comprise entre 75,0 et 175,0 N.s.m⁻² (Pa.s), en combinaison avec un rapport hydro-lipophile moyen à élevé, le rapport hydro-lipophile moyen étant de 6 à 10 et le rapport hydro-lipophile élevé étant de 11 à 25, et une quantité suffisante dudit émulsifiant à viscosité élevée est comprise entre 200 mg et 500 mg, la quantité suffisante de curcumine est comprise entre 5 mg et 50 mg, le rapport de la curcumine à l'émulsifiant à haute viscosité est compris entre 5:95 et 15:85 (p/p), l'acide citrique étant présent selon une teneur comprise entre 1 mg et 10 mg, l'émulsifiant étant le Polysorbate 60.

2. Composition pour l'utilisation selon la revendication 1, qui en outre est encapsulée dans une capsule de gélatine.

3. Composition pour l'utilisation selon les revendications 1 ou 2, comprenant en outre de 3 % (p:p) à 30 % (p:p) d'une huile essentielle comprenant plus de 50 % de composés du terpène.

4. Composition pour l'utilisation selon l'une quelconque des revendications précédentes, comprenant l'émulsifiant à faible viscosité, dans laquelle la quantité suffisante dudit émulsifiant à faible viscosité est comprise entre 600 mg et 700 mg.

5. Composition pour l'utilisation selon l'une quelconque des revendications précédentes, comprenant l'émulsifiant à faible viscosité, dans laquelle la quantité suffisante de la curcumine est comprise entre 30 mg et 50 mg.

6. Composition pour l'utilisation selon l'une quelconque des revendications précédentes, comprenant l'émulsifiant à faible viscosité, dans laquelle le rapport de la curcumine à l'émulsifiant à faible viscosité est compris entre 5:95 et 12:88 (p/p).

7. Composition pour l'utilisation selon l'une quelconque des revendications précédentes, comprenant l'émulsifiant à faible viscosité, dans laquelle l'acide citrique est présent à une concentration comprise entre 2 mg et 5 mg.

8. Composition pour l'utilisation selon l'une quelconque des revendications 1 à 3, comprenant l'émulsifiant à haute viscosité, dans laquelle la quantité suffisante dudit émulsifiant à haute viscosité est comprise entre 300 mg et 500 mg.

9. Composition pour l'utilisation selon l'une quelconque des revendications 1 à 3 ou 8, comprenant l'émulsifiant à haute viscosité, dans laquelle la quantité suffisante de curcumine est comprise entre 10 mg et 30 mg.

10. Composition pour l'utilisation selon l'une quelconque des revendications 1 à 3 ou 8 à 9, comprenant l'émulsifiant à haute viscosité, dans laquelle le rapport de la curcumine à l'émulsifiant à haute viscosité est compris entre 5:95 et 12:88 (p/p).

11. Composition pour l'utilisation selon l'une quelconque des revendications 1 à 3 ou 8 à 10, comprenant l'émulsifiant à haute viscosité, dans laquelle l'acide citrique est présent à une concentration comprise entre 1 mg et 5 mg.

12. Composition pour l'utilisation selon l'une quelconque des revendications 1 à 3 ou 8 à 11, comprenant l'émulsifiant à haute viscosité, comprenant en outre un déshydratant choisi dans le groupe consistant en la cellulose, la méthylcellulose, l'hydroxypropylcellulose et le SiO₂.

13. Composition selon l'une quelconque des revendications précédentes, pour une utilisation dans le traitement ou la prévention d'une inflammation ou de maladies associées à une inflammation.

14. Composition pour l'utilisation selon la revendication 13, pour laquelle la maladie associée à une inflammation est l'ostéoarthrite.
